(19) Europäisches Patentamt
European Patent Office
Office européen des brevets

(11) Numéro de publication: **0 496 653 B1**

(12) # FASCICULE DE BREVET EUROPEEN

(45) Date de publication du fascicule du brevet: **13.09.95**

(51) Int. Cl.6: **A61K 7/13**

(21) Numéro de dépôt: **92400121.7**

(22) Date de dépôt: **17.01.92**

Le dossier contient des informations techniques présentées postérieurement au dépôt de la demande et ne figurant pas dans le présent fascicule.

(54) **Utilisation de dérivés indoliques à titre de coupleurs dans la teinture de fibres kératiniques.**

(30) Priorité: **21.01.91 FR 9100640**

(43) Date de publication de la demande: **29.07.92 Bulletin 92/31**

(45) Mention de la délivrance du brevet: **13.09.95 Bulletin 95/37**

(84) Etats contractants désignés: **AT BE CH DE DK ES FR GB GR IT LI NL PT SE**

(56) Documents cités:
**FR-A- 2 626 771**
**FR-A- 2 649 887**
**GB-A- 2 211 517**
**GB-A- 2 213 169**

(73) Titulaire: **L'OREAL**
**14, rue Royale**
**F-75008 Paris (FR)**

(72) Inventeur: **Cotteret, Jean**
**15, allée des Meuniers**
**F-78480 Verneuil-sur-Seine (FR)**

(74) Mandataire: **Casalonga, Axel et al**
**BUREAU D.A. CASALONGA - JOSSE**
**Morassistrasse 8**
**D-80469 München (DE)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 496 653 B1

Rank Xerox (UK) Business Services
(3.10/3.09/3.3.3)

## Description

La présente invention est relative à l'utilisation de dérivés indoliques à titre de coupleurs pour la teinture des fibres kératiniques et en particulier pour la teinture des cheveux humains, ainsi qu'aux compositions et aux procédés de teinture de mise en oeuvre.

Il est connu de teindre les fibres kératiniques, en particulier les cheveux humains, avec des compositions tinctoriales contenant des précurseurs de colorants d'oxydation et en particulier des p-phénylènediamines, des ortho- ou para-aminophénols appelés généralement "bases d'oxydation".

On sait également qu'on peut faire varier les nuances obtenues avec ces bases d'oxydation en les associant à des coupleurs encore appelés modificateurs de coloration, choisis généralement parmi les métadiamines aromatiques, les méta-aminophénols et les métadiphénols.

La demande de brevet FR-A-2 626 771 décrit l'utilisation d'une association de 5,6-dihydroxyindole ou ses dérivés avec une paraphénylènediamine disubstituée sur l'un des groupes amino pour la teinture progressive des fibres kératiniques.

La demande de brevet GB-A-2 211 517 décrit un procédé de teinture des libres kératiniques en deux étapes mettant en oeuvre l'association de 5,6-dihydroxyindole ou ses dérivés et de colorants d'oxydation rapides ou de coupleurs en présence du système oxydant comportant des ions iodure et du péroxyde d'hydrogène.

On recherche, dans le domaine de la teinture capillaire, des précurseurs de colorants d'oxydation ou des coupleurs permettant de conférer aux cheveux, en milieu oxydant, une coloration ayant une résistance satisfaisante à là lumière, aux lavages, aux intempéries et à la transpiration.

La demanderesse vient de découvrir, ce qui fait l'objet de l'invention, que l'utilisation du 5,6-dihydroxyindole ou de certains de ses dérivés à titre de coupleurs, avec des précurseurs de colorants d'oxydation, permettait d'obtenir après application sur les fibres kératiniques et en particulier les cheveux humains, des teintures aux nuances très variées présentant des résistances à la lumière, aux lavages, aux intempéries et à la transpiration, particulièrement remarquables, notamment lorsqu'ils sont utilisés avec la p-phénylènediamine et ses dérivés. En outre, ces teintures ne tachent pas le cuir chevelu.

Un objet de l'invention est donc constitué par cette utilisation.

L'invention a également pour objet des compositions tinctoriale d'oxydation, destinées à être utilisées pour la teinture des fibres kératiniques, dans laquelle les dérivés indoliques définis ci-après jouent le rôle de coupleurs et contenant au moins un précurseur de colorant d'oxydation du type para et/ou ortho et au moins un agent oxydant.

Un autre objet de l'invention est constitué par le procédé de teinture des fibres kératiniques, en particulier des cheveux humains, mettant en oeuvre ce coupleur.

D'autres objets de l'invention apparaîtront à la lecture de la description et des exemples qui suivent.

Les composés indoliques utilisés comme coupleurs dans la teinture d'oxydation des fibres kératiniques et en particulier des cheveux humains, en présence d'au moins un précurseur de colorant d'oxydation para et/ou ortho, répondent à la formule :

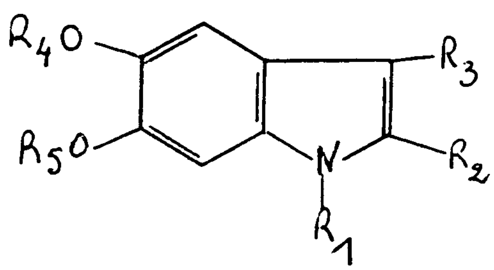

(I)

dans laquelle:

$R_1$ représente un atome d'hydrogène, un groupement alkyle en $C_1$-$C_4$;

$R_2$ et $R_3$, identiques ou différents, représentent un atome d'hydrogène ou un groupement alkyle en $C_1$-$C_4$, un groupement carboxyle ou alcoxycarbonyle en $C_1$-$C_4$;

$R_4$ ou $R_5$, identiques ou différents, représentent un groupement alkyle en $C_1$-$C_4$, un groupement acyle en $C_2$-$C_{20}$, un groupement aryle, ou bien $R_4$ et $R_5$, conjointement avec les atomes d'oxygène et les deux atomes de carbone auxquels ils sont rattachés, forment un cycle contenant éventuellement un groupement

carbonyle, un groupement méthylène, substitué ou non par un ou deux groupements alkyle ou alcoxy ou alkylamino, $R_4$ et $R_5$ pouvant désigner simultanément hydrogène;
et les sels d'addition des acides minéraux ou organiques, ainsi que les sels de métaux alcalins, alcalino-terreux ou d'amines correspondants.

Parmi les composés de formule (I), les composés préférés sont les composés dans lesquels le radical alkyle désigne méthyle, éthyle; le radical alcoxycarbonyle désigne méthoxy ou éthoxycarbonyle; le radical acyloxy désigne acétoxy, tétradécanoyloxy.

Parmi les composés de formule (I) préférés, on peut citer le 5,6-dihydroxyindole, le 2-méthyl 5,6-dihydroxyindole, le 3-méthyl 5,6-dihydroxyindole, le 2,3-diméthyl 5,6-dihydroxyindole, le 1,2-diméthyl 5,6-dihydroxyindole, le 2-éthoxycarbonyl 5,6-dihydroxyindole, le 2,3-diméthyl 5,6-diméthoxyindole, le 5,6-diméthoxyindole, le 5-méthoxy 6-acétoxyindole, le 5,6-dibenzyloxyindole, le 2-carboxy-5,6-dihydroxyindole.

Les précurseurs de colorants de type para ou ortho sont des composés quine sont pas des colorants en eux-mêmes, mais qui forment un colorant par un processus de condensation oxydative, soit sur eux-mêmes, soit en présence d'un coupleur ou modificateur.

Ces composés comportent des groupements fonctionnels, soit deux groupements amino, soit un groupement amino et un groupement hydroxy en position para ou ortho, l'un par rapport à l'autre.

Les précurseurs de type para sont en particulier choisis parmi les paraphénylènediamines, les para-aminophénols, les précurseurs hétérocycliques para, comme la 2,5-diaminopyridine, la 2-hydroxy 5-aminopyridine, la tétraaminopyrimidine, la 4,5 -diamino 1-méthyl pyrazole, la 2-diméthylamino 4,5,6-triaminopyrimidine et les bases dites "doubles".

A titre de paraphénylènediamines, on peut citer les composés répondant à la formule (II) ci-après :

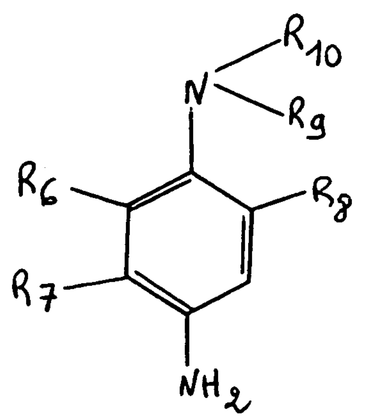

(II)

dans laquelle:
$R_6$, $R_7$ et $R_8$, identiques ou différents, représentent un atome d'hydrogène ou d'halogène, un radical alkyle ayant 1 à 4 atomes de carbone, un radical alcoxy ayant 1 à 4 atomes de carbone;
$R_9$ et $R_{10}$, identiques ou différents, représentent un atome d'hydrogène, un radical alkyle, hydroxyalkyle, alcoxyalkyle, carbamylalkyle, mésylaminoalkyle, acétylaminoalkyle, uréidoalkyle, carbalcoxyaminoalkyle, pipéridinoalkyle, morpholinoalkyle, phényle éventuellement substitué en para par un groupement amino;
ces groupes alkyle ou alcoxy ayant de 1 à 4 atomes de carbone, ou bien $R_9$ et $R_{10}$ forment, conjointement avec l'atome d'azote auquel ils sont liés, un hétérocycle pipéridino ou morpholino, sous réserve que $R_6$ ou $R_8$ représentent un atome d'hydrogène lorsque $R_9$ et $R_{10}$ ne représentent pas un atome d'hydrogène, ainsi que les sels de ces composés.

Parmi les composés de formule (II), on peut citer plus particulièrement la p-phénylènediamine, la p-toluylènediamine, la méthoxyparaphénylènediamine, la chloroparaphénylènediamine, la 2,6-diméthylparaphénylènediamine, la 2,6-diéthylparaphénylènediamine, la 2,5-diméthylparaphénylènediamine, la 2-méthyl 5-méthoxyparaphénylènediamine, la 2,6-diméthyl 5-méthoxyparaphénylènediamine, la N,N-diméthylparaphénylènediamine, la N,N-diéthylparaphénylènediamine, la N,N-dipropylparaphénylènediamine, la 3-méthyl 4-amino N,N-diéthylaniline, la N,N-di($\beta$-hydroxyéthyl)paraphénylènediamine, la 3-méthyl 4-amino N,N-di-($\beta$-hydroxyéthyl)aniline, la 3-chloro 4-amino N,N-di-($\beta$-hydroxyéthyl)aniline, la 4-amino N,N-(éthyl,carbamylméthyl)aniline, la 3-méthyl 4-amino N,N-(éthyl, carbamylméthyl)aniline, la 4-amino N,N-(éthyl,$\beta$-pipéridinoéthyl) aniline, la 3-méthyl 4-amino N,N-(éthyl,$\beta$-pipéridinoéthyl)aniline, la 4-amino N,N-(éthyl,$\beta$-morpholinoéthyl)aniline, la 3-méthyl 4-amino N,N-(éthyl,$\beta$-morpholinoéthyl)aniline, la 4-amino N,N-(éthyl,$\beta$-acétylaminoéthyl)aniline, la 4-amino N-($\beta$-méthoxyéthyl)aniline, la 3-méthyl 4-amino N,N-(éthyl,$\beta$-

acétylaminoéthyl)aniline, la 4-amino N,N-(éthyl,β-mésylaminoéthyl)aniline, la 3-méthyl 4-amino N,N-(éthyl,β-mésylaminoéthyl)aniline, la 4-amino N,N-(éthyl,β-sulfoéthyl)aniline, la 3-méthyl 4-amino N,N-(éthyl,β-sulfoéthyl)aniline, la N-[(4'-amino) phényl]morpholine, la N-[(4'-amino)phényl]pipéridine, la 2-hydroxyéthylparaphénylènediamine, la fluoroparaphénylènediamine, la carboxyparaphénylènediamine, la sulfoparaphénylènediamine, la 2-isopropylparaphénylènediamine, la 2-n-propylparaphénylènediamine, l'hydroxy-2-n-propylparaphénylènediamine, la 2-hydroxyméthylparaphénylènediamine, la N,N-diméthyl 3-méthylparaphénylènediamine, la N,N-(éthyl,β-hydroxyéthyl)paraphénylènediamine, la N-(dihydroxypropyl)paraphénylènediamine, la N-4'-aminophénylparaphénylènediamine, la N-phénylparaphénylènediamine.

Ces précurseurs de colorants par oxydation de type para peuvent être introduits dans la composition tinctoriale, soit sous forme de base libre, soit sous forme de sels, tels que chlorhydrate, bromhydrate ou sulfate.

Parmi les p-aminophénols, on peut citer le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2-hydroxyméthyl 4-aminophénol, le 2-(β-hydroxyéthyl) 4-aminophénol, le 2-méthoxy 4-aminophénol, le 3-méthoxy 4-aminophénol, le 2,5-diméthyl 4-aminophénol, le 2-méthoxyméthyl 4-aminophénol, le 2-aminométhyl 4-aminophénol, le 2-β-hydroxyéthylaminométhyl 4-aminophénol.

Les colorants d'oxydation de type ortho sont choisis parmi les orthoaminophénols, comme le 1-amino 2-hydroxybenzène, le 6-méthyl 1-hydroxy 2-aminobenzène, le 4-méthyl 1-amino 2-hydroxybenzène, les orthophénylènediamines.

Les bases dites doubles sont des bis-phénylènealkylènediamines, répondant à la formule :

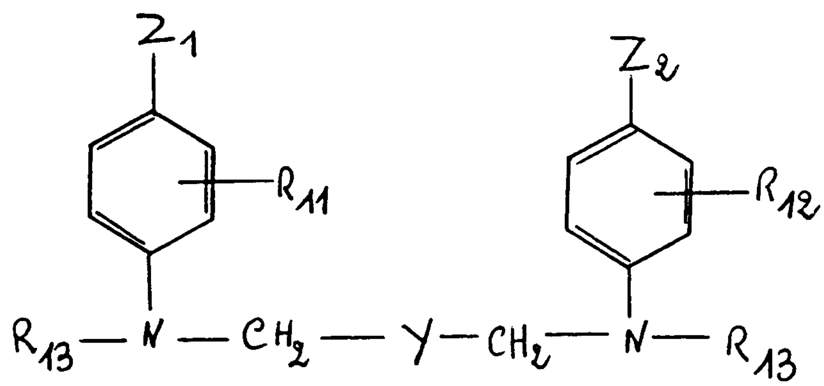

(III)

dans laquelle :

$Z_1$ et $Z_2$, identiques ou différents, représentent des groupements hydroxyle ou $NHR_{14}$, où $R_{14}$ désigne un atome d'hydrogène ou un radical alkyle inférieur;

$R_{11}$ et $R_{12}$, identiques ou différents, représentent, soit des atomes d'hydrogène, soit des atomes d'halogène, soit encore des groupements alkyle;

$R_{13}$ représente un atome d'hydrogène, un groupe alkyle, hydroxyalkyle ou aminoalkyle, dont le reste amino peut être substitué par un ou deux groupements alkyle;

Y représente un radical choisi parmi les radicaux suivants : $-(CH_2)_n-$, $(CH_2)_m-O-(CH_2)_m-$, $-(CH_2)_m-CHOH-(CH_2)_m$

$$-(CH_2)_m-\underset{\displaystyle CH_3}{\underset{|}{N}}-(CH_2)_m;$$

<u>n</u> est un nombre entier compris entre O et 8 et <u>m</u> un nombre entier compris entre O et 4, cette base dite double pouvant se présenter sous forme de ses sels d'addition avec des acides.

Les radicaux alkyle ou alcoxy désignent de préférence un groupement ayant 1 à 4 atomes de carbone et notamment méthyle, éthyle, propyle, méthoxy, éthoxy.

Parmi les composés de formule (III), on peut citer le N,N'-bis-(β-hydroxyéthyl)N,N'-bis-(4'-aminophényl)-1,3-diamino 2-propanol, la N,N'-bis-(β-hydroxyéthyl)N,N'-bis-(4'-aminophényl)éthylènediamine, la N,N'-bis-(4-aminophényl)tétraméthylènediarnine, la N,N′-bis-(β-hydroxyéthyl)N,N′-bis-(4-aminophényl)-tétraméthylènediamine, la N,N′-bis-(4-méthylaminophényl)tétraméthylènediamine, la N,N′-bis-(éthyl) N,N′-bis-(4′-amino 3′-méthylphényl)éthylènediamine.

On peut éventuellement utiliser également en plus du coupleur indolique de formule (I) défini ci-dessus d'autres coupleurs connus en eux-mêmes, tels que les métadiphénols, les métaaminophénols, les métaphénylènediamines, les métaacylaminophénols, les métauréidophénols, les métacarbalcoxyaminophénols, l' α-naphtol, les coupleurs possédant un groupement méthylène actif, tels que les composés β-cétoniques, les pyrazolones, les coupleurs hétérocycliques ou le 4-hydroxyindole, le 6 ou 7-hydroxyindole.

Parmi ces coupleurs, on peut plus particulièrement citer le 2,4-dihydroxyphénoxyéthanol, le 2,4-dihydroxyanisole, le métaaminophénol, le monométhyléther de résorcine, le 2-méthyl 5-N-(β-hydroxy éthyl)-aminophénol, le 2-méthyl 5-N-(β-mésylaminoéthyl)aminophénol, la 6-hydroxybenzomorpholine, le 2,4-diaminoanisole, le 2,4-diaminophénoxyéthanol, la 6-aminobenzomorpholine, le [2-N-(β-hydroxyéthyl) amino 4-amino]-phénoxyéthanol, le 2-amino 4-N-(β-hydroxyéthyl) aminoanisole, le (2,4-diamino)phényl-β,γ-dihydroxypropyléther, la 2,4-diaminophénoxyéthylamine, le 1,3-diméthoxy 2,4-diaminobenzène, le 2-méthyl 5-aminophénol, le 2,6-diméthyl 3-aminophénol, le 1-amino 3,4-méthylènedioxybenzène, le 1-hydroxy 3,4-méthylènedioxybenzène, le 2-chloro 6-méthyl 3-aminophénol, le 2-méthyl 3-aminophénol, le 2-chlororésorcinol, le résorcinol, le 6-méthoxy 3-hydroxyéthylaminoaniline,le 1-éthoxy 2-bis-(β-hydroxyéthyl)amino 4-aminobenzène, le 3-diéthylaminophénol, le 1,3-dihydroxy 2-méthylbenzène, le 1-hydroxy 2,4-dichloro 3-aminobenzène, le 4,6-hydroxyéthoxy 1,3-diaminobenzène, le 4-méthyl 6-éthoxy 1,3-diaminobenzène, le 4-chloro 6-méthyl 3 -aminophénol, le 6-chloro 3-trifluoroéthylaminophénol et leurs sels.

On peut également utiliser conjointement avec les composés précités et comme cela est bien connu dans l'état de la technique, notamment en vue de nuancer ou d'enrichir en reflets les colorations apportées par les précurseurs de colorants d'oxydation et le coupleur de formule (I), des colorants directs, tels que les colorants azoïques, anthraquinoniques ou les dérivés nitrés de la série benzénique.

Lors de la mise en oeuvre du coupleur de formule (I), conformément à l'invention, on n'utilise pas conjointement à ces coupleurs, de dérivé quinonique de la famille des benzoquinones et des naphtoquinones susceptible d'oxyder le composé de formule (I), ni d'ion iodure en quantité susceptible d'oxyder le composé de formule (I) et le précurseur de colorant d'oxydation en présence du coupleur.

L'agent oxydant est choisi de préférence parmi le peroxyde d'hydrogène, le peroxyde d'urée, les bromates de métaux alcalins, les persels, tels que les perborates et les persulfates, le peroxyde d'hydrogène étant particulièrement préféré.

Le coupleur indolique de formule (I) précité est mis en oeuvre à titre de coupleur dans des compositions tinctoriales d'oxydation contenant dans un milieu approprié pour la teinture, en outre au moins un précurseur de colorant d'oxydation para et/ou ortho, ainsi qu'éventuellement les autres coupleurs et colorants directs mentionnés et au moins un agent oxydant.

Ces compositions ne doivent pas contenir d'ion iodure dans des proportions susceptibles d'oxyder le précurseur de colorant d'oxydation et le coupleur de formule (I).

Les compositions tinctoriales qui constituent un autre objet de l'invention sont essentiellement caractérisées par le fait qu'elles contiennent dans un milieu approprié pour la teinture :

a) au moins un coupleur de formule (I)

b) au moins un précurseur de colorant d'oxydation para et/ou ortho choisi parmi les paraaminophénols, les précurseurs hétérocycliques, les orthoaminophénols, les orthophénylènediamines, les bases dites "doubles" telles que définies ci-dessus et les paraphénylènediamines répondant à la formule (IV) :

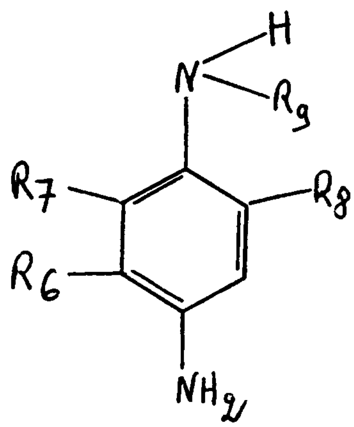

(IV)

dans laquelle:

$R_6$, $R_7$ et $R_8$, identiques ou différents, représentent un atome d'hydrogène ou d'halogène, un radical alkyle ayant de 1 à 4 atomes de carbone, un radical alcoxy ayant de 1 à 4 atomes de carbone;

$R_9$ représente un atome d'hydrogène, un radical alkyle, carbamylalkyle, mésylaminoalkyle, acétylaminoalkyle, uréidoalkyle, carbalcoxyaminoalkyle, pipéridino-alkyle, morpholino-alkyle, ces groupes alkyle ou alcoxy ayant de 1 à 4 atomes de carbone, ainsi que les sels de ces composés.

Parmi les composés de formule (IV), on peut citer la p-phénylènediamine, la p-toluylènediamine, la méthoxyparaphénylènediamine, la chloroparaphénylènediamine, la 2,6-diméthylparaphénylènediamine, la 2,5-diméthylparaphénylènediamine, la 2-méthyl 5-méthoxyparaphénylènediamine, la 2,6-diméthyl 5-méthoxyparaphénylènediamine.

Ces précurseurs de colorants par oxydation de type para peuvent être introduits dans la composition tinctoriale, soit sous forme de base libre, soit sous forme de sels, tels que sous forme de chlorhydrate, de bromhydrate ou de sulfate; et

c) au moins un agent oxydant défini ci-dessus.

Ces compositions ne contiennent pas d'ion iodure dans des quantités susceptibles d'oxyder les précurseurs et les coupleurs en présence.

L'ensemble des précurseurs de colorants par oxydation de type para et/ou ortho, ainsi que les coupleurs utilisés dans les compositions tinctoriales conformes à l'invention, représente de préférence de 0,1 à 10% en poids par rapport au poids total de ladite composition. La concentration en composés indoliques de formule (I) peut varier entre 0,05 et 3,5% en poids par rapport au poids total de la composition.

Le pH de la composition appliquée sur les fibres kératiniques, en particulier les cheveux, a une valeur généralement comprise entre 3 et 11.

Ce pH est ajusté par l'utilisation d'agents acidifiants ou alcalinisants bien connus dans le domaine de la teinture capillaire.

Les compositions tinctoriales conformes à l'invention contiennent généralement, dans leur forme de réalisation préférée, des agents tensio-actifs anioniques, cationiques, non-ioniques, amphotères ou leurs mélanges. Parmi ces agents tensio-actifs, on peut citer les alkylbenzènesulfonates, les alkylnaphtalènesulfonates, les sulfates, les éthersulfates et les sulfonates d'alcools gras, les sels d'ammonium quaternaires, tels que le bromure de triméthylcétylammonium, le bromure de cétylpyridinium; les éthanolamides d'acides gras éventuellement oxyéthylénés; les acides, les alcools ou les amines polyoxyéthylénés, les alcools polyglycérolés, les alkylphénols polyoxyéthylénés ou polyglycérolés, ainsi que les alkylsulfates polyoxyéthylénés.

Ces agents tensio-actifs sont présents dans les compositions utilisées conformément à l'invention, dans des proportions comprises entre 0,5 et 55% en poids, et de préférence entre 2 et 50% en poids, par rapport au poids total de la composition.

Ces compositions peuvent également contenir des solvants organiques pour solubiliser les composés qui ne seraient pas suffisamment solubles dans l'eau.

Parmi ces solvants, on peut citer à titre d'exemple, les alcanols inférieurs en $C_1$-$C_4$, tels que l'éthanol et l'isopropanol; le glycérol; les glycols ou éthers de glycols, comme le 2-butoxyéthanol, l'éthylène glycol, le propylèneglycol, le monoéthyléther et le monométhyléther de diéthylèneglycol, ainsi que les alcools aromatiques comme l'alcool benzylique ou le phénoxyéthanol et les produits analogues ou leurs mélanges.

Les solvants sont présents de préférence dans des proportions comprises entre 1 et 40% en poids, et en particulier entre 5 et 30% en poids par rapport au poids total de la composition.

Les agents épaississants que l'on peut ajouter dans les compositions conformes à l'invention, peuvent être choisis parmi l'alginate de sodium, la gomme arabique, les dérivés de cellulose, les polymères d'acide acrylique, la gomme de xanthane. On peut également utiliser des agents épaississants minéraux tels que la bentonite.

Ces agents épaississants sont présents de préférence dans des proportions comprises entre 0,1 et 5% et en particulier entre 0,2 et 3% en poids par rapport au poids total de la composition.

Les agents antioxydants qui peuvent être présents dans les compositions sont choisis en particulier parmi le sulfite de sodium, l'acide thioglycolique, le bisulfite de sodium, l'acide dihydroascorbique, l'hydroquinone et l'acide homogentisique. Ces agents antioxydants sont présents dans la composition dans des proportions comprises entre 0,05 et 1,5% en poids par rapport au poids total de la composition.

Ces compositions peuvent également contenir d'autres adjuvants cosmétiquement acceptables, tels que par exemple des agents de pénétration, des agents séquestrants, des parfums, des tampons, etc.

Les compositions utilisées conformément à l'invention peuvent se présenter sous des formes diverses, telles que sous forme de liquides, de crèmes, de gels ou sous toute autre forme appropriée pour réaliser une teinture des fibres kératiniques et notamment des cheveux humains. Ces compositions peuvent être conditionnées en flacons aérosols en présence d'un agent propulseur.

Conformément à l'invention, le procédé consiste à appliquer sur les fibres kératiniques une composition préparée au moment de l'emploi contenant au moins un coupleur de formule (I), au moins un précurseur de colorant d'oxydation de type para et au moins un agent oxydant différent des ions iodure dans une quantité suffisante pour développer une coloration.

On utilise de préférence une solution d'eau oxygénée à 20 volumes. Le mélange obtenu est appliqué sur les cheveux et on laisse poser pendant 10 à 40 minutes, de préférence 15 à 30 minutes, après quoi on rince les cheveux, on les lave au shampooing, on les rince à nouveau et on les sèche.

Les composés de formule (I) utilisés de façon préférentielle lorsque la composition appliquée sur les cheveux a un pH inférieur à 9, sont le 5,6-dihydroxyindole, le 2,3-diméthyl 5,6-dihydroxyindole, le 2-méthyl 5,6-dihydroxyindole, le 3-méthyl 5,6-dihydroxyindole, le 1,2-diméthyl 5,6-dihydroxyindole, le 2-carboxy 5,6-dihydroxyindole, le 2-éthoxycarbonyl 5,6-dihydroxyindole, le 5-méthoxy 6-acétoxyindole, le 5,6-dibenzyloxyindole, le 5,6-diméthoxyindole.

Lorsque la composition appliquée sur les cheveux a un pH supérieur à 9, le dérivé indolique est de préférence un dérivé répondant à la formule :

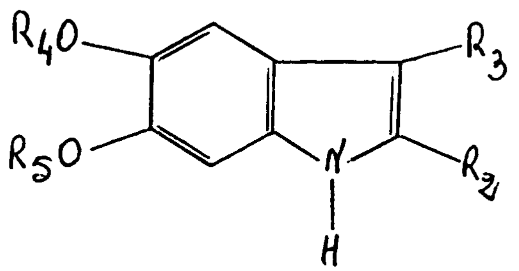

(V)

dans laquelle:

$R_2$ désigne hydrogène, alkyle, alcoxycarbonyle

$R_3$ désigne hydrogène, alkyle

$R_4$ et $R_5$, identiques ou différents, désignent alkyle, acyle, sous réserve que lorsque $R_4$ et $R_5$ désignent alkyle, au moins un groupement $R_2$ et $R_3$ est différent d'hydrogène,

$R_4$ et $R_5$ pouvant désigner conjointement hydrogène.

Parmi les composés de formule (V), on préfere le 5,6-dihydroxyindole, le 2-éthoxycarbonyl 5,6-dihydroxyindole, le 5-méthoxy 6-acétoxyindole.

Une autre forme de réalisation de l'invention consiste à appliquer séparément une composition (A) contenant le coupleur indolique de formule (I) et le précurseur de colorant d'oxydation, puis après rinçage une composition (B) renfermant l'agent oxydant différent des ions iodure.

Il est également possible, conformément à l'invention, d'appliquer séparément les compositions contenant le précurseur de colorant d'oxydation, puis après rinçage, d'appliquer une composition renfermant le coupleur indolique de formule (I) et l'agent oxydant.

Les conditions de pose et de séchage ou de lavage étant similaires à celles indiquées ci-dessus.

Les exemples suivants sont destinés à illustrer l'invention sans pour autant présenter un caractère limitatif.

EXEMPLES 1 à 12

On procède à la teinture des cheveux en appliquant sur des cheveux permanentés ou naturels gris à 90% de blancs un mélange extemporané de la composition colorante (A) et de la composition oxydante (B).

Ce mélange présente le pH indiqué dans les tableaux ci-après. On laisse agir ce mélange pendant 30 minutes, on rince ensuite les cheveux, puis on effectue un shampooing. Après séchage, les cheveux sont teints dans la nuance précisée au bas des tableaux ci-après.

| en g | 1 | 2 | 3 | 4 | 5 | 6 | 7 | 8 |
|---|---|---|---|---|---|---|---|---|
| A) Composition colorante | | | | | | | | |
| 5,6-dihydroxyindole | 0,447 | | | | | | | |
| 2-méthyl 5,6-dihydroxyindole,HBr | | 0,732 | | | | | | |
| 3-méthyl 5,6-dihydroxyindole | | | 0,489 | | | | | |
| 2-carboxy 5,6-dihydroxyindole | | | | 0,579 | | | | |
| 5,6-diméthoxyindole | | | | | 0,532 | | | |
| 2,3-diméthyl 5,6-diméthoxyindole | | | | | | 0,616 | | |
| 1,2-diméthyl 5,6-dihydroxyindole | | | | | | | 0,489 | |
| 5-méthoxy 6-acétoxyindole | | | | | | | | 0,615 |
| Paraphénylènediamine | 0,324 | | | 0,324 | | | 0,324 | |
| 2,6-diméthylparaphénylènediamine,2HCl | | 0,657 | | | 0,657 | 0,657 | | 0,657 |
| Paraaminophénol | | | 0,327 | | | | | |
| Monoéthanolamine qs pH | 9,1 | 8,8 | 9,1 | 8,9 | 8,6 | 9,1 | 9,1 | 8,75 |
| Support 2 | X | X | X | X | X | X | X | X |
| Eau qsp | 100 | 100 | 100 | 100 | 100 | 100 | 100 | 100 |
| | | | | | | | | |
| B) Composition oxydante | | | | | | | | |
| | | | | | | | | |
| Solution d'eau oxygénée à 20 volumes | | | | | | | | |
| Acide phosphorique qs pH | 1,3 | 1,3 | 1,3 | 1,3 | 1,3 | 1,3 | 1,3 | 1,3 |
| pH mélange p/p A+B | 6,7 | 6,2 | 6,7 | 6,7 | 6,1 | 6,7 | 6,7 | 6,4 |
| Nuances obtenues sur cheveux naturels 90% blanc | chatain auburn | cendré mat | blond doré cuivré | naturel acajou irisé | cendré irisé léger. doré | blond beige doré | chatain irisé chaud | chatain clair violine |

| en g | 9 | 10 | 11 | 12 |
|---|---|---|---|---|
| A) Composition colorante | | | | |
| 5,6-dihydroxyindole | 0,149 | | 0,596 | |
| 2-éthoxycarbonyl 5,6-dihydroxyindde | | 0,221 | | |
| 5-méthoxy 6-acétoxyindole | | | | 0,41 |
| Paraphénylènediamine | 0,216 | | | 0,216 |
| 2,6-diméthylparaphénylènediamine,2HCl | | | | |
| Paraaminophénol | | | 0,436 | |
| Métaaminophénol | | 0,109 | | |
| 3-(B-hydroxyéthylamino) 6-méthylphénol | 0,167 | | | |
| 2-méthyl paraphénylènediamine,2HCl | | 0,366 | | |
| α-naphtol | | 0,144 | | |
| Alcool éthylique | 30 | | | |
| Ammoniaque à 20% qs pH | 11,2 | | | |
| pH | | 10,4 | 10,5 | 10,5 |
| Support 1 | | X | X | X |
| Eau qsp | 100 | 100 | 100 | 100 |
| | | | | |
| B) Composition oxydante | | | | |
| | | | | |
| Solution d'eau oxygénée à 20 volumes | | | | |
| Acide phosphorique qs pH | 3 | 3 | 3 | 3 |
| pH mélange p/p 1/3A+ 2/3B | 10 | | 10 | 10 |
| pH mélange p/p A+B | | 9,8 | | |
| Nuances obtenues sur cheveux 90% blanc permanentés | acajou cendré | violet rabattu | marron cuivré | acajou cuivré |

## SUPPORT DE COLORATION 1

| | |
|---|---|
| -Octyldodécanol vendu sous la dénomination d'EUTANOL G par la Société HENKEL | 8,0 g |
| - Acide oléïque | 20,0 g |
| - Lauryléthersulfate de monoéthanolamine vendu sous la dénomination de SIPON LM 35 par la Société HENKEL | 3,0 g |
| - Alcool éthylique | 10,0 g |
| - Alcool benzylique | 10,0 g |
| - Alcool cétylstéarylique oxyéthyléné à 33 moles d'oxyde d'éthylène vendu sous la dénomination de SIMULSOL GS par la Société SEPPIC | 2,4 g |
| - Acide éthylènediamine tétracétique | 0,2 g |
| - Polymère cationique constitué de motifs récurents : 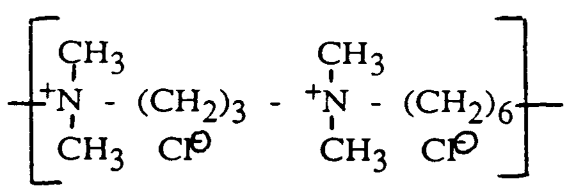 | 2,2 g |
| - Monoéthanolamine | 7,5 g |
| - Diéthanolamide d'acide linoléïque vendu sous la dénomination de COMPERLAN F par la Société HENKEL | 8,0 g |
| - Ammoniaque à 20% | 10,2 g |
| - Métabisulfite de sodium en solution aqueuse à 35% | 0,45 g |
| - Hydroquinone | 0,15 g |
| - 1-phényl 3-méthyl 5-pyrazolone | 0,2 g |

| SUPPORT DE COLORATION 2 | |
|---|---|
| - Alcool oléïque polyglycérolé à 2 moles de glycérol | 4,0 g |
| - Alcool oléïque polyglycérolé à 4 moles de glycérol | 5,71 g MA |
| - Acide oléïque | 3,0 g |
| - Amine oléïque oxyéthyléné à 2 moles d'oxyde d'éthylène vendue sous la dénomination ETHOMEEN O 12 par la Société AKZO | 7,0 g |
| - Laurylaminosuccinamate de diéthylaminopropyle, sel de sodium | 3,0 g MA |
| - Alcool oléïque | 5,0 g |
| - Diéthanolamide d'acide oléïque | 12,0 g |
| - Propylèneglycol | 3,5 g |
| - Alcool éthylique | 7,0 g |
| - Dipropylèneglycol | 0,5 g |
| - Monométhyléther de prolylèneglycol | 9,0 g |
| - Métabisulfite de sodium en solution aqueuse | 0,45 g MA |
| - Acétate d'ammonium | 0,8 g |
| - Antioxydant, séquestrant qs | |

## Revendications

**1.** Utilisation dans la coloration par oxydation des fibres kératiniques, et en particulier des cheveux, mettant en oeuvre des précurseurs de colorants d'oxydation para et/ou ortho en présence d'un agent oxydant choisi parmi le péroxyde d'hydrogène, le péroxyde d'urée, les bromates de métaux alcalins, les persels d'un dérivé indolique répondant à la formule (I) :

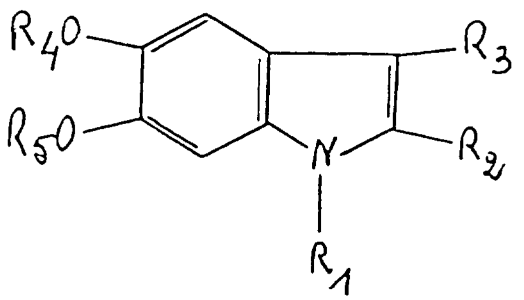

(I)

dans laquelle :
$R_1$ représente un atome d'hydrogène, un groupement alkyle en $C_1$-$C_4$;
$R_2$ et $R_3$, identiques ou différents, représentent un atome d'hydrogène ou un groupement alkyle en $C_1$-$C_4$, un groupement carboxyle ou alcoxycarbonyle en $C_1$-$C_4$;
$R_4$ ou $R_5$, identiques ou différents, représentent un groupement alkyle en $C_1$-$C_4$, un groupement acyle en $C_2$-$C_{20}$, un groupement aryle, ou bien $R_4$ et $R_5$, conjointement avec les atomes d'oxygène et les deux atomes de carbone auxquels ils sont rattachés, forment un cycle contenant éventuellement un groupement carbonyle, un groupement méthylène, substitué ou non par un ou deux groupements alkyle ou alcoxy ou alkylamino, $R_4$ et $R_5$ pouvant désigner simultanément hydrogène;
et les sels d'addition des acides minéraux ou organiques, ainsi que les sels de métaux alcalins, alcalino-terreux ou d'amines correspondants, à titre de coupleur.

**2.** Utilisation selon la revendication 1, caractérisée par le fait que le composé indolique de formule (I) est choisi parmi le 5,6-dihydroxyindole, le 2-méthyl 5,6-dihydroxyindole, le 3-méthyl 5,6-dihydroxyindole, le 2,3-diméthyl 5,6-dihydroxyindole, le 1,2-diméthyl 5,6-dihydroxyindole, le 2-éthoxycarbonyl 5,6-dihydroxyindole, le 2,3-diméthyl 5,6-diméthoxyindole, le 5,6-diméthoxyindole, le 5-méthoxy 6-acétoxyindole, le 5,6-dibenzyloxyindole, le 2-carboxy 5,6-dihydroxyindole.

**3.** Utilisation selon la revendication 1 ou 2, caractérisée par le fait que le précurseur de colorant d'oxydation est choisi parmi les précurseurs de coloration de type para, répondant à la formule :

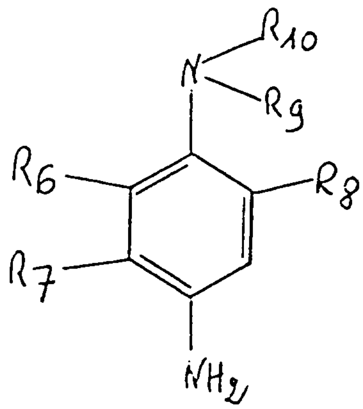

(II)

dans laquelle :
$R_6$, $R_7$ et $R_8$, identiques ou différents, représentent un atome d'hydrogène ou d'halogène, un radical alkyle ayant 1 à 4 atomes de carbone, un radical alcoxy ayant 1 à 4 atomes de carbone;
$R_9$ et $R_{10}$, identiques ou différents, représentent un atome d'hydrogène, un radical alkyle, hydroxyalkyle, alcoxyalkyle, carbamylalkyle, mésylaminoalkyle, acétylaminoalkyle, uréidoalkyle, carbalcoxyaminoalkyle, pipéridinoalkyle, morpholinoalkyle, phényle éventuellement substitué en para par un groupement amino;
ces groupes alkyle ou alcoxy ayant de 1 à 4 atomes de carbone, ou bien $R_9$ et $R_{10}$ forment, conjointement avec l'atome d'azote auquel ils sont liés, un hétérocycle pipéridino ou morpholino, sous réserve que $R_6$ ou $R_8$ représentent un atome d'hydrogène lorsque $R_9$ et $R_{10}$ ne représentent pas un atome d'hydrogène, ainsi que les sels de ces composés.

**4.** Utilisation selon l'une quelconque des revendications 1 à 3, caractérisée par le fait que les précurseurs de colorants d'oxydation sont choisis parmi les paraaminophénols, les orthoaminophénols, les orthophénylènediamines ou les précurseurs hétérocycliques para.

**5.** Utilisation selon l'une quelconque des revendications 1 à 4, caractérisée par le fait que les précurseurs de colorants d'oxydation sont choisis parmi les bases dites "doubles" qui sont des bis-phénylène alkylènediamines, répondant à la formule (III) :

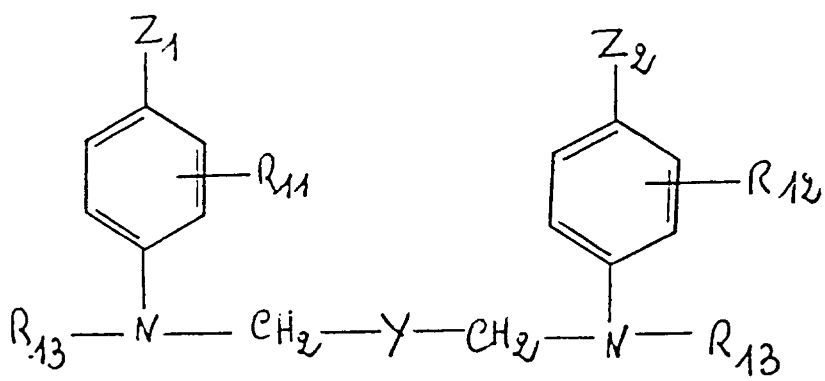

(III)

dans laquelle :
$Z_1$ et $Z_2$, identiques ou différents, représentent des groupements hydroxyle ou $NHR_{14}$, où $R_{14}$ désigne un atome d'hydrogène ou un radical alkyle inférieur;
$R_{11}$ et $R_{12}$, identiques ou différents, représentent, soit des atomes d'hydrogène, soit des atomes d'halogène, soit encore des groupements alkyle;
$R_{13}$ représente un atome d'hydrogène, un groupe alkyle, hydroxyalkyle ou aminoalkyle, dont le reste amino peut être substitué par un ou deux groupements alkyle;
Y représente un radical choisi parmi les radicaux suivants : $-(CH_2)_n-$, $(CH_2)_m-O-(CH_2)_m-$, $-(CH_2)_m-CHOH-(CH_2)_m$

$$-(CH_2)_m-\underset{CH_3}{\underset{|}{N}}-(CH_2)_m;$$

n est un nombre entier compris entre O et 8 et m un nombre entier compris entre O et 4, cette base dite double pouvant se présenter sous forme de ses sels d'addition avec des acides.

**6.** Utilisation selon l'une quelconque des revendications 1 à 5, caractérisée par le fait que l'on met en oeuvre en plus du coupleur indolique de formule (I), d'autres coupleurs.

**7.** Utilisation selon la revendication 6, caractérisée par le fait que les autres coupleurs mis en oeuvre en plus du composé indolique de formule (I) sont choisis parmi les métadiphénols, les méta-aminophénols, les métaphénylènediamines, les méta-acylaminophénols, les méta-uréidophénols, les métacarbalcoxyaminophénols, l'$\alpha$-naphtol, les coupleurs possédant un groupement méthylène actif choisis parmi les composés $\beta$-cétoniques,les pyrazolones, les coupleurs hétérocycliques, le 4-hydroxyindole, le 6-hydroxyindole ou le 7-hydroxyindole.

**8.** Utilisation selon l'une quelconque des revendications 1 à 7, caractérisée par le fait que l'on met en oeuvre en plus, des colorants directs choisis parmi les colorants azoïques, anthraquinoniques ou les dérivés nitrés de la série benzénique.

**9.** Utilisation selon l'une quelconque des revendications 1 à 8, caractérisée par le fait que le composé indolique de formule (I) utilisé à titre de coupleur est mis en oeuvre à l'aide de compositions tinctoriales d'oxydation, contenant dans un milieu approprié pour la teinture, au moins un précurseur de colorant d'oxydation para et/ou ortho, au moins le composé indolique à titre de coupleur de formule (I), ainsi qu'éventuellement d'autres coupleurs et d'autres colorants directs et au moins un agent oxydant différent des ions iodure.

**10.** Composition tinctoriale pour fibres kératiniques, caractérisée par le fait qu'elle contient dans un milieu approprié pour la teinture :

a) au moins un coupleur de formule (I) :

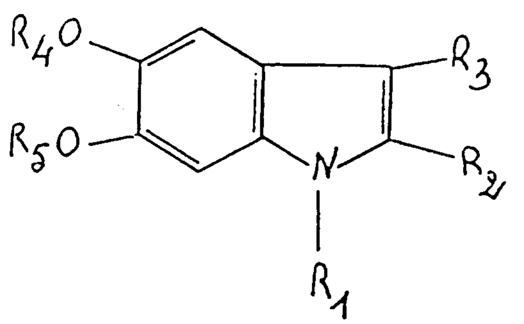

(I)

dans laquelle :

$R_1$ représente un atome d'hydrogène, un groupement alkyle en $C_1$-$C_4$;

$R_2$ et $R_3$, identiques ou différents, représentent un atome d'hydrogène ou un groupement alkyle en $C_1$-$C_4$, un groupement carboxyle ou alcoxycarbonyle en $C_1$-$C_4$;

$R_4$ ou $R_5$, identiques ou différents, représentent un groupement alkyle en $C_1$-$C_4$, un groupement acyle en $C_2$-$C_{20}$, un groupement aryle, ou bien $R_4$ et $R_5$, conjointement avec les atomes d'oxygène et les deux atomes de carbone auxquels ils sont rattachés, forment un cycle contenant éventuellement un groupement carbonyle, un groupement méthylène, substitué ou non par un ou deux groupements alkyle ou alcoxy ou alkylamino, $R_4$ et $R_5$ pouvant désigner simultanément hydrogène;

et les sels d'addition des acides minéraux ou organiques, ainsi que les sels de métaux alcalins, alcalino-terreux ou d'amines correspondants.

b) au moins un précurseur de colorant d'oxydation para et/ou ortho choisi parmi les paraaminophénols, les précurseurs hétérocycliques, les orthoaminophénols, les orthophénylènediamines, les bases dites "doubles" et les paraphénylènediamines répondant à la formule (IV) :

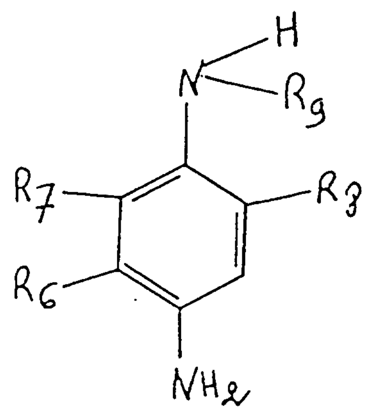

(IV)

dans laquelle :
$R_6$, $R_7$ et $R_8$, identiques ou différents, représentent un atome d'hydrogène ou d'halogène, un radical alkyle ayant de 1 à 4 atomes de carbone, un radical alcoxy ayant de 1 à 4 atomes de carbone;
$R_9$ représente un atome d'hydrogène, un radical alkyle, carbamylalkyle, mésylaminoalkyle, acétylaminoalkyle, uréidoalkyle, carbalcoxyaminoalkyle, pipéridino-alkyle, morpholino-alkyle, ces groupes alkyle ou alcoxy ayant de 1 à 4 atomes de carbone, ainsi que les sels de ces composés.
c) au moins un agent oxydant, cette composition ne contenant pas d'ion iodure.

**11.** Composition selon la revendication 10, caractérisée par le fait que les paraphénylènediamines de formule (IV) sont choisies parmi la p-phénylènediamine, la p-toluylènediamine, la méthoxy paraphénylènediamine, la chloroparaphénylènediamine, la 2,6-diméthylparaphénylènediamine, la 2,5-diméthylparaphénylènediamine, la 2-méthyl 5-méthoxyparaphénylènediamine, la 2,6-diméthyl 5-méthoxyparaphénylènediamine, sous forme de base libre ou sous forme de sels.

**12.** Composition selon l'une quelconque des revendications 10 ou 11, caractérisée par le fait que les précurseurs de colorants d'oxydation de type para et/ou ortho et les coupleurs de formule (I) sont utilisés dans des proportions comprises entre 0,1 et 10% en poids par rapport au poids total de la composition, la concentration en composés indoliques de formule (I) étant comprise entre 0,05 et 3,5% en poids par rapport au poids total de la composition.

**13.** Composition selon l'une quelconque des revendications 10 à 12, caractérisée par le fait que le pH est compris entre 3 et 11.

**14.** Procédé de coloration des fibres kératiniques, en particulier des cheveux, caractérisé par le fait que l'on procède au couplage des précurseurs de colorants d'oxydation de type para, et/ou ortho en présence d'un agent oxydant choisi parmi le péroxyde d'hydrogène, le péroxyde d'urée, les bromates de métaux alcalins, les persels, avec un coupleur répondant à la formule (I) :

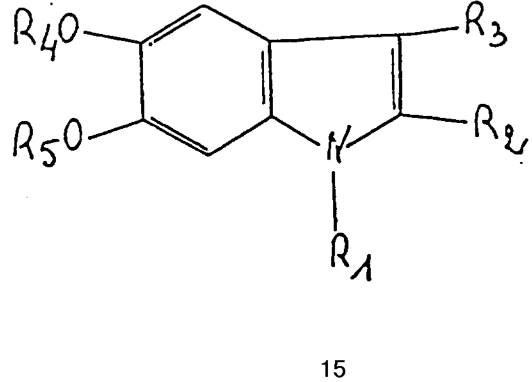

(I)

dans laquelle :
$R_1$ représente un atome d'hydrogène, un groupement alkyle en $C_1$-$C_4$;
$R_2$ et $R_3$, identiques ou différents, représentent un atome d'hydrogène ou un groupement alkyle en $C_1$-$C_4$, un groupement carboxyle ou alcoxycarbonyle en $C_1$-$C_4$;
$R_4$ ou $R_5$, identiques ou différents, représentent un groupement alkyle en $C_1$-$C_4$, un groupement acyle en $C_2$-$C_{20}$, un groupement aryle, ou bien $R_4$ et $R_5$, conjointement avec les atomes d'oxygène et les deux atomes de carbone auxquels ils sont rattachés, forment un cycle contenant éventuellement un groupement carbonyle, un groupement méthylène, substitué ou non par un ou deux groupements alkyle ou alcoxy ou alkylamino, $R_4$ et $R_5$ pouvant désigner simultanément hydrogène;
et les sels d'addition des acides minéraux ou organiques, ainsi que les sels de métaux alcalins, alcalino-terreux ou d'amines correspondants.

**15.** Procédé selon la revendication 14, caractérisé par le fait que l'on applique une composition préparée au moment de l'emploi contenant au moins un coupleur de formule (I), au moins un précurseur de colorant d'oxydation de type para et/ou ortho et au moins un agent oxydant, sur les fibres kératiniques, en particulier sur les cheveux, qu'on laisse poser pendant 10 à 40 minutes cette composition, après quoi on rince les cheveux, on les lave au shampooing, on les rince à nouveau et on les sèche.

**16.** Utilisation selon l'une quelconque des revendications 1 à 9, caractérisée par le fait que l'on procède à la teinture à un pH inférieur à 9.

**17.** Utilisation selon la revendication 16, caractérisée par le fait que le coupleur est choisi parmi le 5,6-dihydroxyindole, le 2,3-diméthyl 5,6-dihydroxyindole, le 2-méthyl 5,6-dihydroxyindole, le 3-méthyl 5,6-dihydroxyindole, le 1,2-diméthyl 5,6-dihydroxyindole, le 2-carboxy 5,6-dihydroxyindole, le 2-éthoxycarbonyl 5,6-dihydroxyindole, le 5-méthoxy 6-acétoxyindole, le 5,6-dibenzyloxyindole, le 5,6-diméthoxyindole.

**18.** Utilisation selon l'une quelconque des revendications 1 à 9, caractérisée par le fait que l'on procède à la teinture des fibres kératiniques à un pH supérieur à 9, le dérivé indolique de formule (I) étant choisi parmi les composés de formule (V) :

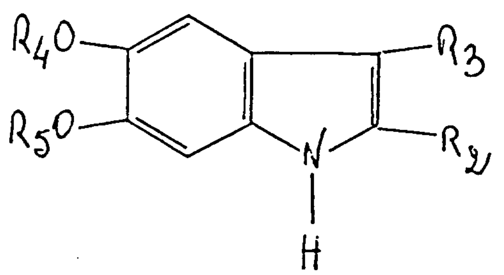

(V)

dans laquelle :
$R_2$ désigne hydrogène, alkyle, alcoxycarbonyle
$R_3$ désigne hydrogène, alkyle
$R_4$ et $R_5$, identiques ou différents, désignent alkyle, acyle,
sous réserve que lorsque $R_4$ et $R_5$ désignent alkyle, au moins un groupement $R_2$ et $R_3$ est différent d'hydrogène, $R_4$ et $R_5$ pouvant désigner conjointement hydrogène.

**19.** Procédé de teinture des fibres kératiniques, en particulier des cheveux humains, caractérisé par le fait que l'on applique séparément, d'une part une composition (A) contenant le coupleur indolique tel que défini dans l'une quelconque des revendications 1 et 2, et un précurseur de colorant d'oxydation para et/ou ortho tel que défini dans l'une quelconque des revendications 1 à 5, éventuellement un autre coupleur, puis après rinçage, une composition (B) renfermant un agent oxydant choisi parmi le péroxyde d'hydrogène, le péroxyde durée les bromates de métaux alcalins, les persels, les compositions (A) et (B) étant exemptes d'ions iodure.

**20.** Procédé de teinture des fibres kératiniques, en particulier des cheveux, caractérisé par le fait que l'on applique séparément une composition (A) renfermant un précurseur de colorant d'oxydation para et/ou ortho tel que défini dans l'une quelconque des revendications 1 à 5, puis après rinçage, une composition (B) renfermant un composé indolique de formule (I) et l'agent oxydant choisi parmi le péroxyde d'hydrogène, le péroxyde durée, les bromates de métaux alcalins, les persels.

## Claims

**1.** Use in the oxidation dyeing of keratinous fibres, and in particular of hair, using para and/or ortho oxidation dye precursors in the presence of an oxidizing agent chosen from hydrogen peroxide, urea peroxide, alkali metal bromates and persalts of an indole derivative corresponding to the formula (I):

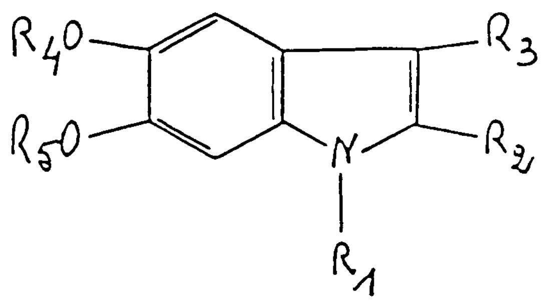

(I)

in which:
$R_1$ denotes a hydrogen atom or a $C_1$-$C_4$ alkyl group,
$R_2$ and $R_3$, which are identical or different, denote a hydrogen atom or a $C_1$-$C_4$ alkyl group or a carboxyl or $C_1$-$C_4$ alkoxycarbonyl group,
$R_4$ and $R_5$, which are identical or different, denote a $C_1$-$C_4$ alkyl group, a $C_2$-$C_{20}$ acyl group, an aryl group, or else $R_4$ and $R_5$, together with the oxygen atoms and the two carbon atoms to which they are attached, form a ring optionally containing a carbonyl group or a methylene group unsubstituted or substituted by one or two alkyl or alkoxy or alkylamino groups, it being possible for $R_4$ and $R_5$ simultaneously to denote hydrogen,
and the addition salts of inorganic or organic acids, as well as the corresponding alkali, alkaline-earth metal or amine salts,

**2.** Use according to Claim 1, characterized in that the indole compound of formula (I) is chosen from 5,6-dihydroxyindole, 2-methyl-5,6-dihydroxyindole, 3-methyl-5,6-dihydroxyindole, 2,3-dimethyl-5,6-dihydroxyindole, 1,2-dimethyl-5,6-dihydroxyindole, 2-ethoxycarbonyl-5,6-dihydroxyindole, 2,3-dimethyl-5,6-dimethoxyindole, 5,6-dimethoxyindole, 5-methoxy-6-acetoxyindole, 5,6-dibenzyloxyindole and 2-carboxy-5,6-dihydroxyindole.

**3.** Process according to Claim 1 or 2, characterized in that the oxidation dye precursor is chosen from the dye precursors of para type, corresponding to the formula:

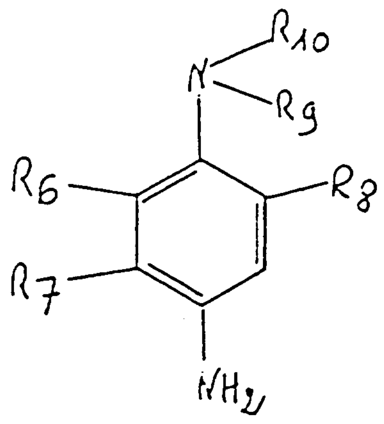

(II)

in which:

$R_6$, $R_7$ and $R_8$, which are identical or different, denote a hydrogen or halogen atom, an alkyl radical containing 1 to 4 carbon atoms, or an alkoxy radical containing 1 to 4 carbon atoms,

$R_9$ and $R_{10}$, which are identical or different, denote a hydrogen atom or an alkyl, hydroxyalkyl, alkoxyalkyl, carbamylalkyl, mesylaminoalkyl, acetylaminoalkyl, ureidoalkyl, carbalkoxyaminoalkyl, piperidinoalkyl, morpholinoalkyl or phenyl radical optionally substituted by an amino group in the para position,

these alkyl or alkoxy groups containing from 1 to 4 carbon atoms, or else $R_9$ and $R_{10}$, together with the nitrogen atom to which they are bonded, form a piperidino or morpholino heterocylic ring, provided tht $R_6$ and $R_8$ denote a hydrogen atom when $R_9$ and $R_{10}$ do not denote a hydrogen atom, and the salts of these compounds.

**4.** Use according to any one of Claims 1 to 3, characterized in that the oxidation dye precursors are chosen from para-aminophenols, ortho-aminophenols, ortho-phenylenediamines or para-heterocyclic precursors.

**5.** Use according to any one of Claims 1 to 4, characterized in that the oxidation dye precursors are chosen from so-called "double" bases which are bis-phenylenealkylenediamines corresponding to the formula (III):

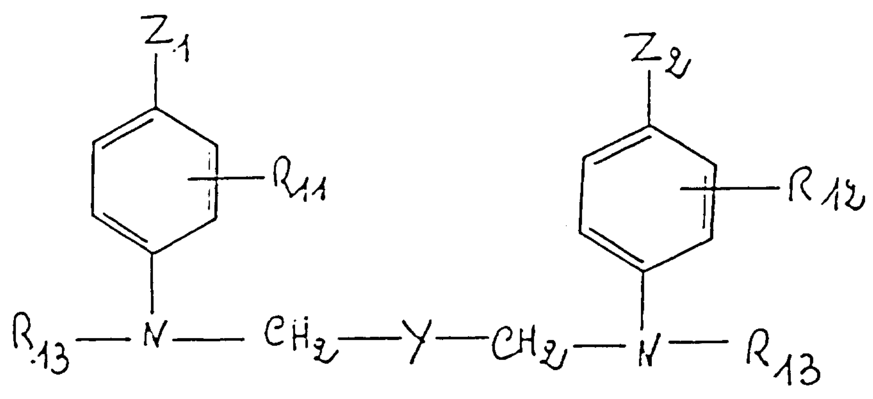

(III)

in which:

$Z_1$ and $Z_2$, which are identical or different, denote hydroxyl or $NHR_{14}$ groups where $R_{14}$ denotes a hydrogen atom or a lower alkyl radical,

$R_{11}$ and $R_{12}$, which are identical or different, denote either hydrogen atoms or halogen atoms or else alkyl groups,

$R_{13}$ denotes a hydrogen atom or an alkyl, hydroxyalkyl or aminoalkyl group in which the amino residue may be substituted by one or two alkyl groups,

Y denotes a radical chosen from the following radicals: $-(CH_2)_n-$, $(CH_2)_m-O-(CH_2)_m-$, $-(CH_2)_m-CHOH-(CH_2)_m$

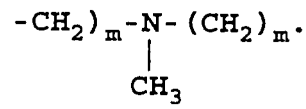

n is an integer between 0 and 8 and m an integer between 0 and 4, it being possible for this so-called double base to be in the form of its addition salts with acids.

**6.** Use according to any one of Claims 1 to 5, characterized in that other couplers are used in addition to the indole coupler of formula (I).

**7.** Use according to Claim 7, characterized in that the other couplers used in addition to the indole coupler of formula (I) are chosen from meta-diphenols, meta-aminophenols, meta-phenylenediamines, meta-acylaminophenols, meta-ureidophenols, meta-carbalkoxyaminophenols, $\alpha$-napthol, couplers containing an active methylene group which are chosen from $\beta$-ketonic compounds, pyrazolones, heterocyclic couplers, 4-hydroxyindole, 6-hydroxyindole and 7-hydroxyindole.

**8.** Use according to any one of Claims 1 to 7, characterized in that, in addition, direct dyes chosen from azo or anthraquinone dyes or nitro derivatives of the benzene series are used.

**9.** Use according to any one of Claims 1 to 8, characterized in that the indole compound of formula (I) employed as coupler is used with the aid of oxidation dye compositions containing, in a medium which is suitable for dyeing, at least one para and/or ortho oxidation dye precursor, at least the indole compound as coupler of formula (I), and optionally other couplers and other direct dyes and at least one oxidizing agent other than iodide ions.

**10.** Dye composition for keratinous fibres, characterized in that it contains in a medium appropriate for dyeing:

a) at least one coupler of formula (I):

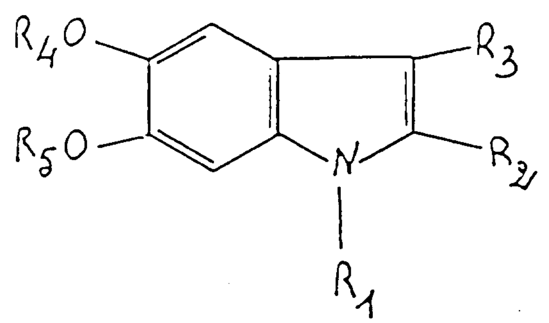

(I)

in which:

$R_1$ denotes a hydrogen atom or a $C_1$-$C_4$ alkyl group,

$R_2$ and $R_3$, which are identical or different, denote a hydrogen atom or a $C_1$-$C_4$ alkyl group or a carboxyl or $C_1$-$C_4$ alkoxycarbonyl group,

$R_4$ and $R_5$, which are identical or different, denote a $C_1$-$C_4$ alkyl group, a $C_2$-$C_{20}$ acyl group, an aryl group, or else $R_4$ and $R_5$, together with the oxygen atoms and the two carbon atoms to which they are attached, form a ring optionally containing a carbonyl group or a methylene group unsubstituted or substituted by one or two alkyl or alkoxy or alkylamino groups, it being possible for $R_4$ and $R_5$ simultaneously to denote hydrogen,

and the addition salts of inorganic or organic acids, as well as the corresponding alkali, alkaline-earth metal or amine salts;

b) at least one para and/or ortho oxidation dye precursor chosen from para-aminophenols, heterocylic precursors, ortho-aminophenols, ortho-phenylenediamines, so-called "double" bases and para-phenylenediamines corresponding to the formula (IV):

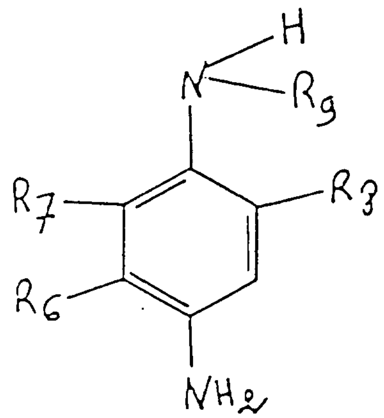

(IV)

in which:

$R_6$, $R_7$ and $R_8$, which are identical or different, denote a hydrogen or halogen atom, an alkyl radical containing from 1 to 4 carbon atoms or an alkoxy radical containing from 1 to 4 carbon atoms,

$R_9$ denotes a hydrogen atom or an alkyl, carbamylalkyl, mesylaminoalkyl, acetylaminoalkyl, ureidoalkyl, carbalkoxyaminoalkyl, piperidinoalkyl or morpholinoalkyl radical, these alkyl or alkoxy groups containing from 1 to 4 carbon atoms, and the salts of these compounds;

c) at least one oxidizing agent, this composition containing no iodide ion.

**11.** Composition according to Claim 10, characterized in that the para-phenylenediamines of formula (IV) are chosen from p-phenylenediamine, p-tolylenediamine, methoxy-para-phenylenediamine, chloro-para-phenylenediamine, 2,6-dimethyl-para-phenylenediamine, 2,5-dimethyl-para-phenylenediamine, 2-methyl-5-methoxy-para-phenylenediamine and 2,6-dimethyl-5-methoxy-para-phenylenediamine, in the form of free base or in the form of salts.

**12.** Composition according to either of Claims 10 and 11, characterized in that the oxidation dye precursors of para and/or ortho type and the couplers of formula (I) are employed in proportions of between 0.1 and 10 % by weight relative to the total weight of the composition, the concentration of indole compounds of formula (I) being between 0.05 and 3.5 % by weight relative to the total weight of the composition.

**13.** Composition according to any one of Claims 10 to 12, characterized in that the pH is between 3 and 11.

**14.** Process for dyeing keratinous fibres, in particular hair, characterized in that oxidation dye precursors of para type are coupled, in the presence of an oxidizing agent chosen from hydrogen peroxide, urea peroxide, alkali metal bromates and persalts, with a coupler corresponding to the formula (I):

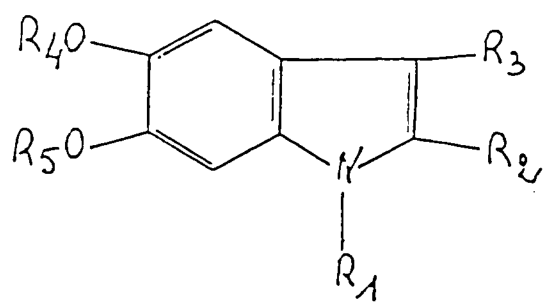

(I)

in which:

$R_1$ denotes a hydrogen atom or a $C_1$-$C_4$ alkyl group,

$R_2$ and $R_3$, which are identical or different, denote a hydrogen atom or a $C_1$-$C_4$ alkyl group or a carboxyl or $C_1$-$C_4$ alkoxycarbonyl group,

$R_4$ and $R_5$, which are identical or different, denote a $C_1$-$C_4$ alkyl group, a $C_2$-$C_{20}$ acyl group, an aryl group, or else $R_4$ and $R_5$, together with the oxygen atoms and the two carbon atoms to which they are attached, form a ring optionally containing a carbonyl group or a methylene group unsubstituted or substituted by one or two alkyl or alkoxy or alkylamino groups, it being possible for $R_4$ and $R_5$ simultaneously to denote hydrogen,
and the addition salts of inorganic or organic acids, as well as the corresponding alkali, alkaline-earth metal or amine salts.

**15.** Process according to Claim 14, characterized in that a composition prepared at the time of use, containing at least one coupler of formula (I), at least one oxidation dye precursor of para type and at least one oxidizing agent is applied to the keratinous fibres, in particular to hair, that this composition is left in place for 10 to 40 minutes, after which the hair is rinsed, washed with shampoo, rinsed again and dried.

**16.** Use according to any one of Claims 1 to 9, characterized in that the dyeing is performed at a pH lower than 9.

**17.** Use according to Claim 16, characterized in that the coupler is chosen from 5,6-dihydroxyindole, 2, 3-dimethyl-5,6-dihydroxyindole, 2-methy-5,6-dihydroxyindole, 3-methyl-5,6-dihydroxyindole, 1,2-dimethyl-5,6-dihydroxyindole, 2-carboxy-5,6-dihydroxyindole, 2-ethoxycarbonyl-5,6-dihydroxyindole, 5-methoxy-6-acetoxyindole, 5,6-dibenzyloxyindole and 5,6-dimethoxyindole.

**18.** Use according to any one of Claims 1 to 9, characterized in that keratinous fibres are dyed at a pH higher than 9, the indole derivative of formula (I) being chosen from the compounds of formula (V):

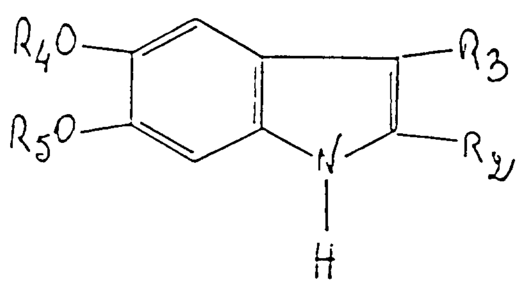

(V)

in which:
$R_2$ denotes hydrogen, alkyl or alkoxycarbonyl,
$R_3$ denotes hydrogen or alkyl,
$R_4$ and $R_5$, which are identical or different, denote alkyl or acyl,
provided that when $R_4$ and $R_5$ denote alkyl, at least one group $R_2$ and $R_3$ is other than hydrogen, it being possible for $R_4$ and $R_5$ jointly to denote hydrogen.

**19.** Process for dyeing keratinous fibres, in particular human hair, characterized in that there are applied separately, on the one hand, a composition (A) containing the indole coupler as defined in either of Claims 1 and 2, and a para and/or ortho oxidation dye precursor as defined in any one of Claims 1 to 5, optionally another coupler and then, after rinsing, a composition (B) containing an oxidizing agent chosen from hydrogen peroxide, urea peroxide, alkali metal bromates and persalts, the compositions (A) and (B) being free from iodide ions.

**20.** Process for dyeing keratinous fibres, in particular hair, characterized in that there are applied separately a composition (A) containing a para and/or ortho oxidation dye precursor as defined in any one of Claims 1 to 5 and then, after rinising, a composition (B) containing an indole compound of formula (I) and the oxidizing agent chosen from hydrogen peroxide, urea peroxide, alkali metal bromates and persalts.

**Patentansprüche**

1. Verwendung eines Indolderivats der Formel (I):

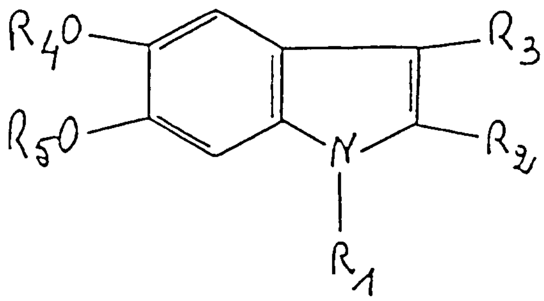

(I)

worin gilt:
$R_1$ stellt ein Wasserstoffatom oder eine $C_{1-4}$-Alkylgruppe dar;
$R_2$ und $R_3$ stellen, gleich oder verschieden, ein Wasserstoffatom oder eine $C_{1-4}$-Alkyl-, Carboxyl- oder $C_{1-4}$-Alkoxycarbonylgruppe dar;
$R_4$ oder $R_5$ stellen, gleich oder verschieden, eine $C_{1-4}$-Alkyl-, $C_{2-20}$-Acyl- oder Arylgruppe dar, oder $R_4$ und $R_5$ bilden zusammen mit den Sauerstoffatomen, an die sie gebunden sind, einen gegebenenfalls eine Carbonylgruppe enthaltenden Ring oder eine Methylengruppe, die mit einer oder zwei Alkyl- oder Alkoxy- oder Alkylaminogruppen substituiert ist oder nicht, wobei $R_4$ und $R_5$ gleichzeitig auch Wasserstoff bedeuten können,
und von Additionssalzen davon mit minderalischen oder organischen Säuren sowie von entsprechenden Alkali-, Erdalkali- oder Aminsalzen davon
als Kuppler bei der Oxidationsfärbung keratinischer Fasern, insbesondere der Haare, wobei man Oxidationsfarbstoff-Vorstufen vom para- und/oder ortho-Typ in Gegenwart eines oxidierenden Mittels zur Anwendung bringt, das aus Wasserstoffperoxid, Harnstoffperoxid, Alkalibromaten und Persalzen ausgewählt ist.

2. Verwendung gemäß Anspruch 1,
dadurch **gekennzeichnet**, daß
die Indolverbindung der Formel (I) aus 5,6-Dihydroxyindol, 2-Methyl-5,6-dihydroxyindol, 3-Methyl-5,6-dihydroxyindol, 2,3-Dimethyl-5,6-dihydroxyindol, 1,2-Dimethyl-5,6-dihydroxyindol, 2-Ethoxycarbonyl-5,6-dihydroxyindol, 2,3-Dimethyl-5,6-dimethoxyindol, 5,6-Dimethoxyindol, 5-Methoxy-6-acetoxyindol, 5,6-Dibenzyloxyindol und aus 2-Carboxy-5,6-dihydroxyindol ausgewählt ist.

3. Verwendung gemäß Anspruch 1 oder 2,
dadurch **gekennzeichnet**, daß
die Oxidationsfarbstoff-Vorstufe aus den Färbungsvorstufen vom para-Typ der Formel:

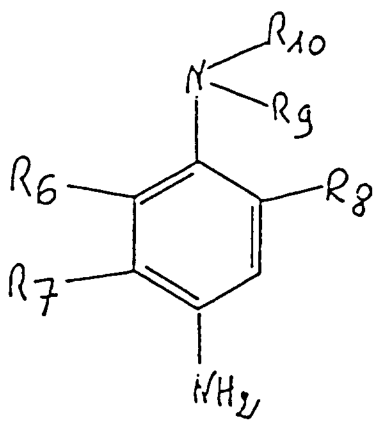

(II)

worin gilt:
$R_6$, $R_7$ und $R_8$ stellen, gleich oder verschieden, ein Wasserstoff- oder Halogenatom, einen Alkylrest mit 1 bis 4 Kohlenstoffatomen oder einen Alkoxyrest mit 1 bis 4 Kohlenstoffatomen dar;
$R_9$ und $R_{10}$ stellen, gleich oder verschieden, ein Wasserstoffatom, einen Alkyl-, Hydroxyalkyl-, Alkoxyalkyl-, Carbamylalkyl-, Mesylaminoalkyl-, Acetylaminoalkyl-, Ureidoalkyl-, Carbalkoxyaminoalkyl-, Piperidinoalkyl-, Morpholinoalkyl- oder Phenylrest dar, der gegebenenfalls in para-Stellung mit einer Aminogruppe substituiert ist, wobei diese Alkyl- oder Alkoxygruppen 1 bis 4 Kohlenstoffatome aufweisen, oder $R_9$ und $R_{10}$ bilden, zusammen mit dem Stickstoffatom, an das sie gebunden sind, auch einen Piperidino- oder Morpholino-Heterozyklus, mit der Maßgabe, daß $R_6$ oder $R_8$ ein Wasserstoffatom darstellen, wenn $R_9$ und $R_{10}$ kein Wasserstoffatom darstellen,
sowie aus den Salzen dieser Verbindungen ausgewählt sind.

4. Verwendung gemäß jedem der Ansprüche 1 bis 3,
dadurch **gekennzeichnet**, daß
die Oxidationsfarbstoff-Vorstufen aus p-Aminophenolen, o-Aminophenolen, o-Phenylendiaminen oder heterozyklischen Vorstufen vom para-Typ ausgewält sind.

5. Verwendung gemäß jedem der Ansprüche 1 bis 4,
dadurch **gekennzeichnet**, daß
die Oxidationsfarbstoff-Vorstufen aus als "doppelte" bezeichneten Basen ausgewählt und Bisphenylenalkylendiamine der Formel (III) sind:

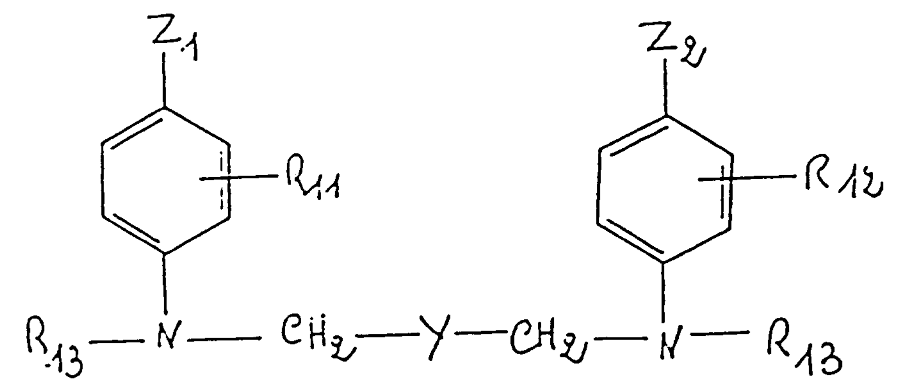

(III)

worin gilt:
$Z_1$ und $Z_2$ stellen, gleich oder verschieden, Hydroxyl- oder $NHR_{14}$-Gruppen dar, worin $R_{14}$ ein Wasserstoffatom oder einen Niedrigalkylrest bedeutet;
$R_{11}$ und $R_{12}$ stellen, gleich oder verschieden, entweder Wasserstoffatome oder Halogenatome oder auch Alkylgruppen dar;
$R_{13}$ stellt ein Wasserstoffatom, eine Alkyl-, Hydroxyalkyl- oder Aminoalkylgruppe dar, deren Aminorest mit einer oder zwei Alkylgruppen substituiert sein kann;
Y stellt einen Rest dar, der aus den folgenden Resten ausgewählt ist:
$-(CH_2)_n-$, $(CH_2)_m-O-(CH_2)_m-$, $-(CH_2)_m-CHOH-(CH_2)_m$

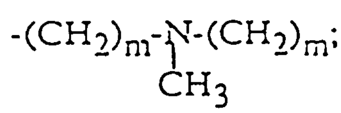

n ist eine ganze Zahl von 0 bis 8, und m ist eine ganze Zahl von 0 bis 4,
wobei diese als doppelte bezeichnete Base in Form ihrer Additionssalze mit Säuren vorliegen kann.

6. Verwendung gemäß jedem der Ansprüche 1 bis 5,
dadurch **gekennzeichnet**, daß
man zusätzlich zum Indol-Kuppler der Formel (I) weitere Kuppler zur Anwendung bringt.

**7.** Verwendung gemäß Anspruch 6,
dadurch **gekennzeichnet**, daß
die zusätzlich zur Indolverbindung der Formel (I) angewandten weiteren Kuppler aus m-Diphenolen, m-Aminophenolen, m-Phenylendiaminen, m- Acylaminophenolen, m-Ureidophenolen, m-Carbalkoxyphenolen, $\alpha$-Naphthol, aus Kupplern mit einer aktiven Methylengruppe, ausgewählt aus $\beta$-Ketoverbindungen, Pyrazolonen und heterozyklischen Kupplern, aus 4-Hydroxyindol, 6-Hydroxyindol oder 7-Hydroxyindol ausgewählt sind.

**8.** Verwendung gemäß jedem der Ansprüche 1 bis 7,
dadurch **gekennzeichnet**, daß
man zusätzlich Direktfarbstoffe zur Anwendung bringt, ausgewählt aus Azofarbstoffen, Antrachinonfarbstoffen oder aus nitrierten Derivaten der Benzolreihe.

**9.** Verwendung gemäß jedem der Ansprüche 1 bis 8,
dadurch **gekennzeichnet**, daß
die als Kuppler verwendete Indolverbindung der Formel (I) mittels Oxidiationsfärbezusammensetzungen eingesetzt wird, die in einem zur Färbung geeigneten Milieu mindestens eine Oxidationsfarbstoff-Vorstufe vom para- und/oder ortho-Typ, mindestens die Indolverbindung der Formel (I) als Kuppler sowie gegebenenfalls weitere Kuppler und weitere Direktfarbstoffe und mindestens ein von Jodidionen verschiedenes oxidierendes Mittel enthalten.

**10.** Färbezusammensetzung für keratinische Fasern,
dadurch **gekennzeichnet**, daß
sie in einem zur Färbung geeigneten Milieu:
a) mindestens einen Kuppler der Formel (I):

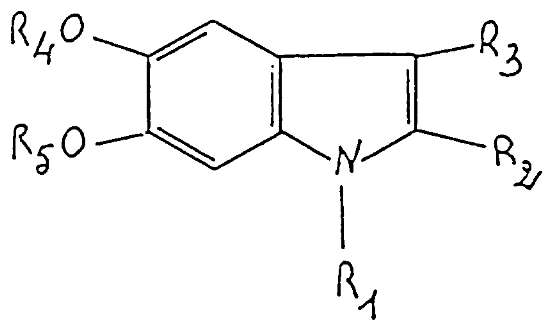

(I)

worin gilt:
$R_1$ stellt ein Wasserstoffatom oder eine $C_{1-4}$-Alkylgruppe dar;
$R_2$ und $R_3$ stellen, gleich oder verschieden, ein Wasserstoffatom oder eine $C_{1-4}$-Alkyl-, Carboxyl- oder $C_{1-4}$-Alkoxycarbonylgruppe dar;
$R_4$ oder $R_5$ stellen, gleich oder verschieden, eine $C_{1-4}$-Alkyl-, $C_{2-20}$-Acyl- oder Arylgruppe dar, oder $R_4$ und $R_5$ bilden zusammen mit den Sauerstoffatomen, an die sie gebunden sind, einen gegebenenfalls eine Carbonylgruppe enthaltenden Ring oder eine Methylengruppe, die mit einer oder zwei Alkyl- oder Alkoxy- oder Alkylaminogruppen substituiert ist oder nicht, wobei $R_4$ und $R_5$ gleichzeitig auch Wasserstoff bedeuten können,
und deren Additionssalze mit mineralischen oder organischen Säuren sowie deren entsprechende Alkali-, Erdalkali- oder Aminsalze,
b) mindestens eine Oxidationsfarbstoff-Vorstufe vom para- und/oder ortho-Typ, ausgewählt aus p-Aminophenolen, heterozyklischen Vorstufen, o-Aminophenolen, o-Phenylendiamin, den als "doppelte" bezeichneten oben definierten Basen und aus p-Phenylendiaminen der Formel (IV):

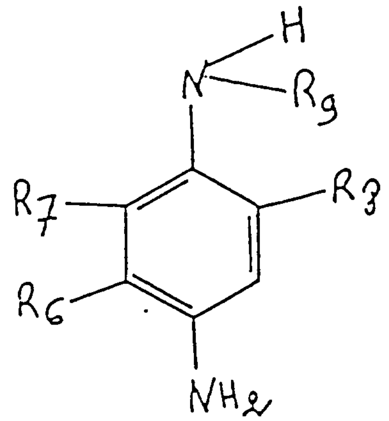

(IV)

worin gilt:
$R_6$, $R_7$ und $R_8$ stellen, gleich oder verschieden, ein Wasserstoff- oder Halogenatom, einen Alkylrest mit 1 bis 4 Kohlenstoffatomen oder einen Alkoxyrest mit 1 bis 4 Kohlenstoffatomen dar;
$R_9$ stellt ein Wasserstoffatom, einen Alkyl-, Carbamylalkyl-, Mesylaminoalkyl-, Acetylaminoalkyl-, Ureidoalkyl-, Carbalkoxyaminoalkyl-, Piperidinoalkyl- oder Morpholinoalkylrest dar, wobei die Alkyl- oder Alkoxygruppen 1 bis 4 Kohlenstoffatome aufweisen;
sowie die Salze dieser Verbindungen und
c) mindestens ein oxidierendes Mittel enthält, wobei diese Zusammensetzung kein Jodidion aufweist.

**11.** Zusammensetzung gemäß Anspruch 10,
dadurch **gekennzeichnet**, daß
die p-Phenylendiamine der Formel (IV) aus p-Phenylendiamin, p-Toluylendiamin, Methoxy-p-phenylendiamin, Chlor-p-phenylendiamin, 2,6-Dimethyl-p-phenylendiamin, 2,5-Dimethyl-p-phenylendiamin, 2-Methyl-5-methoxy-p-phenylendiamin, 2,6-Dimethyl-5-methoxy-p-phenylendiamin, in Form der freien Base oder in Form von Salzen, ausgewählt sind.

**12.** Zusammensetzung gemäß jedem der Ansprüche 10 oder 11,
dadurch **gekennzeichnet**, daß
die Oxidationsfarbstoff-Vorstufen vom para- und/oder ortho-Typ und die Kuppler der Formel (I) in Mengenanteilen von 0,1 bis 10 Gew.%, bezogen auf das Gesamtgewicht der Zusammensetzung, verwendet werden, wobei die Konzentration an Indolverbindungen der Formel (I) 0,05 bis 3,5 Gew.% beträgt, bezogen auf das Gesamtgewicht der Zusammensetzung.

**13.** Zusammensetzung gemaß jedem der Ansprüche 10 bis 12,
dadurch **gekennzeichnet**, daß
der pH-Wert 3 bis 11 beträgt.

**14.** Verfahren zur Färbung keratinischer Fasern, insbesondere der Haare,
dadurch **gekennzeichnet**, daß
man Oxidationsfarbstoff-Vorstufen vom para-Typ in Gegenwart eines aus Wasserstoffperoxid, Harnstoffperoxid, Alkalibromaten und Persalzen ausgewählten oxidierenden Mittels mit einem Kuppler der Formel (I):

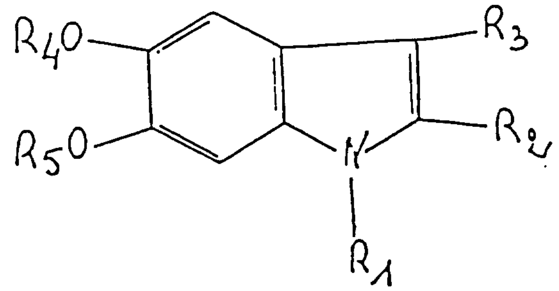

(I)

worin gilt:
$R_1$ stellt ein Wasserstoffatom oder eine $C_{1-4}$-Alkylgruppe dar;
$R_2$ und $R_3$ stellen, gleich oder verschieden, ein Wasserstoffatom oder eine $C_{1-4}$-Alkyl-, Carboxyl- oder $C_{1-4}$-Alkoxycarbonylgruppe dar;
$R_4$ oder $R_5$ stellen, gleich oder verschieden, eine $C_{1-4}$-Alkyl-, $C_{2-20}$-Acyl- oder Arylgruppe dar, oder $R_4$ und $R_5$ bilden zusammen mit den Sauerstoffatomen, an die sie gebunden sind, einen gegebenenfalls eine Carbonylgruppe enthaltenden Ring oder eine Methylengruppe, die mit einer oder zwei Alkyl- oder Alkoxy- oder Alkylaminogruppen substituiert ist oder nicht, wobei $R_4$ und $R_5$ gleichzeitig auch Wasserstoff bedeuten können,
und mit deren Additionssalzen mit mineralischen oder organischen Säuren sowie mit deren entsprechenden Alkali-, Erdalkali- oder Aminsalzen kuppelt.

**15.** Verfahren gemäß Anspruch 14,
dadurch **gekennzeichnet**, daß
man eine zum Zeiptunkt der Anwendung zubereitete Zuammensetzung, die mindestens einen Kuppler der Formel (I), mindestens eine Oxidationsfarbstoff-Vorstufe vom para-Typ und mindestens ein oxidierendes Mittel enthält, auf die keratinischen Fasern, insbesondere die Haare, aufbringt, diese Zusammensetzung 10 bis 40 Minuten lang verweilen läßt, worauf man die Haare spült, sie unter Schamponieren wäscht, erneut spült und dann trocknet.

**16.** Verwendung gemäß jedem der Ansprüche 1 bis 9,
dadurch **gekennzeichnet**, daß
man die Färbung bei einem pH-Wert von weniger als 9 durchführt.

**17.** Verwendung gemäß Anspruch 16,
dadurch **gekennzeichnet**, daß
der Kuppler aus 5,6-Dihydroxyindol, 2,3-Dimethyl-5,6-dihydroxyindol, 2-Methyl-5,6-dihydroxyindol, 3-Methyl-5,6-dihydroxyindol, 1,2-Dimethyl-5,6-dihydroxyindol, 2-Carboxy-5,6-dihydroxyindol, 2-Ethoxycarbonyl-5,6-dihydroxyindol, 5-Methoxy-6-acetoxyindol, 5-6-Dibenzyloxyindol, 5,6-Dimethoxyindol ausgewählt ist.

**18.** Verwendung gemäß jedem der Ansprüche 1 bis 9,
dadurch **gekennzeichnet**, daß
man die Färbung der keratinischen Fasern bei einem pH-Wert von mehr als 9 durchführt, wobei das Indolderivat der Formel (I) aus Verbindungen der Formel (V) ausgewählt ist:

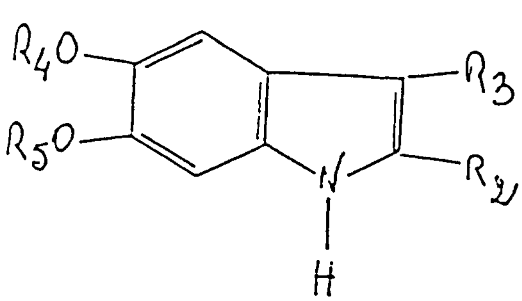

(V)

worin gilt:
$R_2$ bedeutet Wasserstoff, einen Alkyl- oder Alkoxycarbonylrest,
$R_3$ bedeutet Wasserstoff oder einen Alkylrest,
$R_4$ und $R_5$ bedeuten, gleich oder verschieden, einen Alkyl- oder Acylrest, mit der Maßgabe, daß, wenn $R_4$ und $R_5$ einen Alkylrest bedeuten, mindestens eine Gruppe $R_2$ und $R_3$ verschieden von Wasserstoff ist,
wobei $R_4$ und $R_5$ gleichzeitig auch Wasserstoff bedeuten können.

**19.** Verfahren zur Färbung keratinischer Fasern, insbesondere der menschlichen Haare,
dadurch **gekennzeichnet**, daß
man, getrennt voneinander, einerseits eine Zusammensetzung (A), enthaltend den in jedem der Ansprüche 1 und 2 definierten Indol-Kuppler und eine in jedem der Ansprüche 1 bis 5 definierte Oxidationsfarbstoff-Vorstufe vom para- und/oder ortho-Typ sowie gegebenenfalls einen weiteren Kuppler, und dann nach Spülung eine Zusammensetzung (B) aufbringt, die ein aus Wasserstoffperoxid, Harnstoffperoxid, Alkalibromaten und Persalzen ausgewähltes oxidierendes Mittel enthält, wobei die Zusammensetzungen (A) und (B) frei von Jodidionen sind.

**20.** Verfahren zur Färbung keratinischer Fasern, insbesondere der Haare,
dadurch **gekennzeichnet**, daß
man, getrennt voneinander, eine Zusammensetzung (A), die eine in jedem der Ansprüche 1 bis 5 definierte Oxidationsfarbstoff-Vorstufe vom para- und/oder ortho-Typ enthält, und nach Spülung eine Zusammensetzung (B) aufbringt, die eine Indolverbindung der Formel (I) und das aus Wasserstoffperoxid, Harnstoffperoxid, Alkalibromaten und Persalzen ausgewählte oxidierende Mittel enthält.